Europäisches Patentamt

(19) European Patent Office    (11) Publication number: **0 138 338**

Office européen des brevets    **B1**

(12)    EUROPEAN PATENT SPECIFICATION

(45) Date of publication of patent specification: **16.09.87**    (51) Int. Cl.⁴: **C 12 N 11/02,** C 07 D 499/42,
C 07 D 501/18 // C12R1:11

(21) Application number: **84305798.5**

(22) Date of filing: **24.08.84**

(54) Penicillinamidase.

(30) Priority: **12.09.83 US 531537**

(43) Date of publication of application:
**24.04.85 Bulletin 85/17**

(45) Publication of the grant of the patent:
**16.09.87 Bulletin 87/38**

(84) Designated Contracting States:
**DE FR IT NL**

(56) References cited:
**AT-B- 340 859**
**DE-A-2 849 764**
**DE-B-2 336 829**
**IN-A- 139 908**
**US-A-3 736 231**

(73) Proprietor: **AMERICAN HOME PRODUCTS
CORPORATION
685, Third Avenue
New York, New York 10017 (US)**

(72) Inventor: **Cross, Philip Judson
79 White Clay Crescent Newark
New Castle Delaware (US)**

(74) Representative: **Brown, Keith John Symons
et al
c/o John Wyeth & Brother Limited Patent and
Trademark Department Huntercombe Lane
South
Taplow Maidenhead Berkshire SL6 0PH. (GB)**

# 0 138 338

**Description**

This invention relates to pencillinamidase, in particular to a penicillinamidase adduct.

Insolubilization of enzymes by immobilization on the surface of various support materials, both organic and inorganic, is a widely used technique that permits removal and reuse of the otherwise soluble protein. The pure or partially pure enzyme may be immobilized by physical methods such as adsorption, entrapment and microencapsulation or by chemical methods involving ionic or covalent bonding to a vast variety of insolubilizing materials.

An illustrative general approach to enzyme precipitation is disclosed in U.S. 3,736,231 in which an aqueous dispersion of enzyme is treated with a large excess of tannic acid to form an insoluble enzyme-tannic acid complex which is subsequently treated with an excess of glutaraldehyde to obtain an enzyme-tannic acid glutaraldehyde mixture which is separated from the diluent by centrifugation. The product may be used directly, particularly if it is granular or crystalline although the patentee prefers to mix the complex with an inert carrier or support such as diatomaceous earth, etc. for use in packed columns.

Penicillinamidase from both *Escherichia coli* and *Bacillus megaterium* has been immobilized by a number of methods which generally involve either encapsulation of the enzyme within or immobilization onto the surface of cells or other inert support materials. The intracellular enzyme for *E. coli* has been immobilized *in situ* by treatment of whole microbial cells with glutaraldehyde to form cell aggregates. A variation on this theme is disclosed in Indian Patent 139908 in which cells of E. coli were disrupted and the penicillinamidase was partially purified and the cytoplasmic protein was cross-linked with glutaraldehyde to afford a flocculent precipitate. It has also been entrapped within synthetic fibers after costly cell disruption and enzyme purification procedures. The extracellular enzyme produced by *B. megaterium* has been immobilized by adsorption onto bentonite. Pencillinamidase from both organisms has been immobilized on the surface of *Pencillin* mycelium and *Bacillus megaterium* cells (DE—A—2849764).

Glutaraldehyde, as well as other bifunctional crosslinking agents, has been used widely to immobilize enzymes onto solid, inert supports; to form soluble polymers of enzymes that can be immobilized in inert gels; and to form highly cross-linked protein gels. Glutaraldehyde has also been used to immobilize intracellular enzymes by immobilizing the cells of the microorganisms that produce them. Glutaraldehyde is the reagent of choice for linking enzymes to support material because excessive denaturation and inactivation of the protein does not occur.

The use of solid supports to insolubilize an enzyme reduces the activity volume ratio of the product because of the presence of roughly 90 to 99.9 percent inert material which limits the specific activity of such preparations. In addition, an enzyme adsorbed on a solid support covers the support in patches, presenting a mono-layer or at most several layers anchored by weak physical forces which permit resolubilization of enzyme during use with consequential loss of catalytic activity. Enzymes covalently bonded to a support are more securely held, but frequently suffer marked loss of activity during the chemical reaction linking them to the support as a result of denaturation, blockage of active sites or reaction with the active site with the support surface groups, thereby reducing the activity of the enzyme. Surface layers of enzyme immobilized on a solid support are also subject to inactivation by surface active agents, fatty acid esters and other substances that can physically cover the active sites of the enzyme.

Treatment of whole microbial cells with glutaraldehyde to immobilize or stabilize the enzyme in the cell and to form aggregates of cells so that flocs of a desired size are obtained, are limited in the activity that can be achieved because the cells contain large amounts of inert cell material. The capacity of the microbe to produce the enzyme also limits the specific activity of the immobilized aggregates. In this case the enzyme is more protected against the action of inactivating agents than in surface immobilization. Consequently, the life of such preparations is usually longer. The prime disadvantage resides in increased diffusional resistance for both the substrate and product thus slowing down the reaction velocity considerably in practical application.

This invention particularly concerns a solid, particulate, water insoluble, intermolecularly crosslinked penicillin amidase-dialdehyde adduct in which said dialdehyde contains from 5 to 7 carbon atoms, a method for producing said adduct and a process for producing a 6-amino-pencillanic acid, 7-aminocephalosporanic acid and 7-aminodesacetoxy-cephalosporanic acid employing said adduct as a catalyst. More specifically the invention provides an immobilized, intermolecular adduct of the exocellular penicillinamidase produced by *Bacillus megaterium* and an aliphatic dialdehyde containing from 5 to 7 carbon atoms.

The amidase employed in the production of the self-immobilized adduct of this invention is obtained from *Bacillus megaterium*, a representative strain of which is *B. megaterium* ATCC 14945.

The choice of *Bacillus megaterium* as the source of pencllinamidase is advantageous for several reasons. The enzyme is produced as an exocellular enzyme and as such is secreted into the broth in which the organism is grown. The penicillinamidase is only one of a few exocellular proteins produced by strains of *Bacillus megaterium* but it is present in large quantities relative to the other exocellular proteins. Concentration of fermentation broth further increases the proportion of penicillinamidase by removing some of the small molecular weight proteins thus enabling very high specific activities to be obtained in the insolubilized preparation. Comparable activities are difficult to achieve with organisms such as *Escherichia coli*, which produce an intracellular penicillinamidase because it is present along with many other cellular

2

proteins thus diluting the specific activity. In order to increase the specific activity of the enzyme from *E. coli,* the cells must be broken and the enzyme purified by complicated and costly procedures.

The dialdehyde employed as a cross-linking agent in the self-immobilization of penicillinamidase may be a readily available dialdehyde such as glutaraldehyde (1,5...pentanedial), adipaldehyde (1,6 dexanedial) or 1,7-heptanedial. Of these dialdehydes, glutaraldehyde is preferred because it is economical and minimizes denaturation of the enzyme during cross-linking.

The pencillinamidase-dialdehyde adduct exhibits chemical and physical properties suitable for repeated long term use in the production of 6-aminopenicllanic acid (6-APA) by hydrolysis of various natural penicllins, preferably benzylpenicillin (Pen G) or phenoxymethylpenicillin (Pen V) or to form 7-aminocephalosporanic acid (7-ACA) or 7-amino-desacetoxy-cephalosporanic acid (7-ADCA) by hydrolyzing benzylcephalosporin C or 3-methyl-7-phenoxyacetamide-$\Delta^3$-cephem-4-carboxylate, and analogous derivatives.

The penicillinamidase-dialdehyde adduct of this invention has no additional support. The enzyme-dialdehyde product is self-supporting and undergoes very slow loss of activity by enzyme degradation during its use. The structure imparts good mechanical properties to the enzyme particles that makes it possible to use them in enzyme reactors of various types, such as stirred reactors, fluidized beds, or columns to achieve the above mentioned reactions in a batch, semicontinuous or continuous operation with easy enzyme removal from the reaction solution by sedimentation and decantation, by filtration or by centrifugation. Because no inactive (inert) support materials are used during immobilization, the specific activity of the immobilized enzyme is much higher. This allows much smaller volume amounts of the catalyst to be used in the reactions thus reducing suspended solids content and viscosity and allows wider choice of reactor types and configurations.

Where the specific activity of an enzyme preparation is low such as in the prior art immobilized systems, large volume amounts of the catalyst must be used to keep the duration of the reaction in a practical range, especially in cases where the substrate or product (like penicillin or 6-APA) is unstable and degraded to undesirable byproducts under the conditions necessary to perform the reaction. To shorten the reaction times and improve the yield, large amounts of conventional immobilized enzymes have to be used, exceeding the volume of the reaction solution. The only practical method in this case is to pass the solution through a bed or a column filled with the enzyme. This has several disadvantages. The penicillin conversion to 6-APA is accompanied by a pH change. The acidity of the solution increases as it passes through the bed of the enzyme which brings the pH below the optimum range for enzyme activity and the reaction slows down or stops. For that reason the bed height can only be short and the extent of conversion in one pass is limited to a small fraction of the original substrate concentration. Consequently, the substrate pH must be adjusted and the liquid recirculated until a sufficient conversion yield is obtained. This results in complicated types of equipment with multiple columns in parallel or in series and with multiple pH adjustments, pumps, and valves. The pH change on the column reduces the efficiency of the enzyme by operating it outside the optimum. The enzyme in a column cannot be packed absolutely uniformly. This results in channeling which reduces the efficiency of the column in that area where a portion of the enzyme is not supplied with fresh substrate and the product is not removed. This again causes a drop in pH and inactivation of the enzyme. High flows necessary to minimize the pH drop on the column cause packing of the enzyme. The rise in pressure thus produced creates leaks, hazardous conditions, and excessive power consumption for liquid circulation.

The high specific activity of the self-immobilized enzyme of this invention permits use of less than 10% by volume of the enzyme relative to the volume of substrate solution [usually 9—12% (w/v)] which can be converted in less than one hour (usually 30—45 min). This enables use of the self-immobilized enzyme in fluidized form in the substrate solution by the simple means of stirring or gas sparging. Very low energy input is needed to keep the particles suspended in the solution. This does not mean that the self-immobilized enzyme could not be used in packed beds and columns, but the operation in fluidized form is simpler and less energy demanding. It has the advantage that all enzyme particles are in intimate contact with the substrate solution, no channeling exists, and the stirring reduces diffusional resistances to the substrate and product mass transfer. The pH and temperature control is simple and the pH can be kept exactly at the optimum with little fluctuation. No high pressure operation is required and there are no sealing, pumping, or valve problems. After completion of the reaction the enzyme can be removed by simple sedimentation and decantation or by filtration or centrifugation. Short reaction times reduce the losses so that yields of 98% of theory can be achieved.

The immobilized preparation of this invention has advantages of both surface immobilized and encapsulated enzymes. By virtue of the procedure employed in the production of the amidase-dialdehyde adduct, the immobilized enzyme forms particles composed of only the desired enzyme molecules cross-linked by the dialdehyde, one to another in the form of a multilayer network so that the substrate can easily diffuse in the products out and diffusion is not as limiting as in cases of aggregates of cross-linked whole cells. The outer layer of the immobilized enzyme of this invention functions as a protective barrier for the inner layers so that inactivation of more than the surface layer is reduced or eliminated. Even if the outer layer is inactivated, the inner enzyme will function in a manner similar to the encapsulated form. This results in longer life in the presence of inactivating impurities. Because the entire particle is composed of enzyme which is to a large degree accessible to the substrate, the specific activity is extremely high and

3

limited only by the purity of the enzyme used for immobilization. The insolubilized enzyme exhibits between 4500 and 5600 International Units per gram (I.U./g) dry weight of benzylpenicillin hydrolytic activity or 9,500 to 12,000 I.U./g. protein.

The method for producing the penicillinamidase dialdehyde adduct represents an additional aspect of this invention. In essence, the process comprises adding an aliphatic dialdehyde containing 5 to 7 carbon atoms to an aqueous solution containing the exocellular penicillinamidase produced by *Bacillus megaterium*, at a pH of between about 6 to about 9.5 to obtain a final dialdehyde concentration of about 0.1 to about 2.0 weight/volume percent, at a temperature of from 0 to about 45°C, followed by the addition of a multivalent anion containing salt. Preferably the temperature is maintained between 0 and 30°C, e.g. about 15 to about 25°C. Ambient temperature is more preferable.

More specifically, the self-immobilized penicillin-amidase-dialdehyde adduct may be prepared, by way of example, in the following manner. *Bacillus megaterium* is grown in fermentation medium supplemented with phenylacetic acid (PAA) or similar compounds to induce the production of the exocellular enzyme penicillinamidase which is secreted into the fermentation medium. After the bacterial cells are removed from the fermentation medium by flocculation, filtration and/or centrifugation, the enzyme is concentrated by ultrafiltration with 10,000—100,000 molecular weight cut-off filters until the broth contains generally 8—16 mg/ml protein and 30—300 I.U. of penicillinamidase per milliliter. After the ultrafiltered enzyme concentrate is centrifuged or filtered to remove most of the remaining bacterial cells, the pH is adjusted to between 6 and 9 and the enzyme is insolubilized by the addition of dialdehyde (glutaraldehyde is preferred) to a final concentration of 0.1 to 2.0% (w/v). The dialdehyde is allowed to react with the ultrafiltered concentrate for 5 to 60 minutes at 0 to 30 degrees C. After reacting with the dialdehyde, a 0.5M solution of a multivalent anion such as phosphate, thiosulfate, borate, carbonate or sulfate, preferably employed as an alkali metal (sodium or potassium) or ammonium salt, is added to the dialdehyde treated enzyme and allowed to mix for 5 to 60 minutes. The addition of phosphate, thiosulfate, borate, carbonate or sulfate to the dialdehyde treated enzyme solution is required to obtain an insolubilized enzyme with the proper characteristics, e.g. non-gelatinous and able to be retained by a filter. Addition of the anion either before or coincident with the dialdehyde results in a poor floc which is not retained during filtration. The insolubilized enzyme is then collected by either centrifugation or filtration and washed with 2—4 volumes of $H_2O$ or a free aldehyde blocking reagent such as 0.1M glycine or similar compounds containing free primary amino groups at about pH7 to about pH9. It can then be used, for example, for hydrolysis of Pen G to form 6-APA and phenyl-acetic acid (PAA), hydrolysis of Pen V to form 6-APA and phenoxyacetic acid (POAA), or hydrolysis of benzylcephalosporin C to form 7-ACA and PAA after it is resuspended in a suitable reaction mixture.

In accordance with an additional aspect of this invention, there is provided a process for the production of 6-APA, 7-ACA or 7-ADCA which comprises incubating an aqueous solution of a natural penicillin, or a cephalosporin containing a 7-acyl group corresponding to the 6-acyl group of a natural penicillin with the penicillin-amidase-dialdehyde adduct of the present invention. The incubation may be carried out at a temperature of from about 15 to about 42°C. for a time sufficient to remove said acyl groups.

The process of this invention, employing the penicillinamidase-dialdehyde adduct as a catalyst in the production of 6-APA, is less expensive and faster than chemical processes currently in use. The immobilized enzyme of this invention exhibits extremely high activity and stability, which permits short conversion times and continuous recycling of the enzyme for many conversion cycles. As a result, the process and production facility employed in the production of 6-APA may be kept much simpler and is less expensive than that necessary for production of 6-APA with commercially available insolubilized penicillinamidase preparations.

A reaction mixture for hydrolysis of Pen G, Pen V, benzylcephalosporin C or its 7-phenoxymethyl-3-desacetoxy analogue might be, for example, water or an aqueous buffered solution at pH 5 to 12 acetate, citrate, phosphate, TES (N-Tris-[hydroxymethyl]methyl-2-aminoethanesulfonic acid), borate, Tris (Tris-[hydroxymethyl]aminomethane), bicarbonate, carbonate, or a mixture of two or more of these at a concentration of 0.05 to 0.5 moles/liter and containing either Pen G, Pen V, benzylcephalosporin C or 7-phenoxymethyl-3-desacetoxy-cephalosporin at a concentration of 0.5 to 12 grams/dl. Typically, the reaction mixture for activity testing consists of 0.05 M sodium tetraborate, pH 8.5 containing 3% (w/v) Pen G. The reaction mixture containing insolubilized enzyme is then incubated at 15 to 42 degrees C for various periods of time. The initial reaction rate of the enzyme is determined by measuring the amount of 6-APA (in the case of Pen G or Pen V) 7-ACA (in the case of benzylcephalosporin C) or 7-ADCA (in the case of the phenoxymethyl-3-desacetoxy cephalosporin) present in the reaction mixture and is expressed as units/ml where 1 units equals 1 micromole of 6-APA, 7-ACA or 7-ADCA produced per hour or International Units/ml where 1 International Unit (I.U.) equals 1 micromole of 6-APA, 7-ACA or 7-ADCA produced per minute.

The reaction rate and yield of 6-APA, 7-ACA or 7-ADCA may be increased significantly during the reaction by incorporating an anion exchange resin, such as AG1—X8, AG2—X8, Dowex® 1—X8 and Amberlite® IRA—400, into the reaction mixture to remove the hydrolysis products solution.

The following examples illustrate the process for producing the immobilized enzyme of this invention and its use in the removal of acyl groups from penicillin and cephalosporin.

Example 1
Preparation of insolubilized penicillinamidase

The penicillinamidase produced by *Bacillus megaterium* ATCC 14945 [J. Bact. *93* 302 (1967)] is concentrated approximately 35 to 120 times by ultrafiltration with 20,000—100,000 molecular weight cutoff filters. After centrifugation to remove any cells and debris, the enzyme in the concentrated broth was insolubilized.

50 ml of concentrated broth at 12.7 mg/ml protein containing 132.9 I.U./ml of penicillinamidase was adjusted to a pH of 7.0 with dilute HCl. While stirring the concentrate, 3 ml of an 8% (w/v) solution of glutaraldehyde in water was added. After gently stirring at ambient temperature for 30 minutes, 5 ml of 0.5 M sodium phosphate buffer, pH 7.0 was added to the preparation and stirring was continued for an additional 30 minutes at ambient temperature. The insolubilized product resulting from this treatment was collected on a paper filter and washed with 4 volumes of water. The washed product was scraped off of the filter paper and resuspended by vigorous mixing in water at a concentration of 0.1 g wet weight/1 ml of water. The insolubilized penicillinamidase retained 15.3% of the original Pen G hydrolytic activity of the original broth concentrate and had 597.6 I.U./gww and 3619.0 I.U./gdw measured as initial reaction rate when 3% (w/v) Pen G was used as a substrate.

Example 2
Effect of phosphate on yield of insolubilized penicillinamidase

Concentrated broth from the fermentation of *Bacillus megaterium* was produced as described in Example 1. The eznyme in 50 ml aliquots of this concentrated broth was adjusted to pH 7.0 and then insolubilized by the addition of 3 ml of 8% glutaraldehyde. 5 ml of 0.5 M sodium phosphate, pH 7.0 was added to the concentrated broth either before the addition of glutaraldehyde, 30 minutes after the addition of glutaraldehyde, or not at all. The insolubilized penicillinamidase was collected on filter paper, washed with water, and analyzed for Pen G hydrolytic activity. The total grams wet weight (gww) and percent of the initial activity present in each of the insolubilized preparations are presented in Table 1.

TABLE 1

| Time phosphate added | gww | Activity % yield | Relative yield |
|---|---|---|---|
| None added | 1.8 | 9.3 | 49.5% |
| Before glutaraldehyde | 0.4 | 1.3 | 6.9% |
| After glutaraldehyde | 1.5 | 18.8 | 100.0% |

Example 3
Hydrolysis of benzylpenicillin, phenoxymethylpenicillin, and benzylcephalosporin C with insolubilized penicillinamidase

Insolubilized penicillinamidase was produced in a manner similar to that described in Example 1. This preparation was assayed for its ability to produce 6-amino-penicillanic acid from hydrolysis of benzylpenicillin and phenoxymethylpenicillin, or 7-aminocephalosporanic acid from benzylcephalosporin C. Various amounts of insolubilized penicillinamidase were incubated at 37 degrees C. in a solution of 0.1 M sodium tetraborate, pH 8.5 containing 3 g/dl of either Pen G, Pen V, or benzylcephalosporin C and samples were removed periodically for assay of 6-APA or 7-ACA content. The results are expressed as International Units (I.U.) per gram wet weight or gram dry weight of insolubilized enzyme and are listed in Table 2.

TABLE 2

| Substrate | I.U./gww | I.U./gdw |
|---|---|---|
| Benzylpenicillin | 733 | 4213 |
| Phenoxymethylpenicillin | 25 | 143 |
| Benzylcephalosporin C | 58 | 331 |

Example 4
Large-scale insolubilization of penicillin amidase

A pool of 15 liters of concentrated broth containing penicillinamidase from 8 fermentations conducted in a manner similar to that described in Example 1 were adjusted to pH 7.0 by the addition of concentrated HCl and placed in a 20 liter fermentation tank. Insolubilization of the penicillinamidase was performed by the addition of 568 ml of 13.2% glutaraldehyde with vigorous agitation at ambient temperature. After 30 minutes, 1.5 liters of 0.5 M sodium phosphate, pH 7.0 was added and the preparation was agitated for an additional 30 minutes. The insolublized penicillinamidase was collected by filtration and washed with 30 liters of water. The Pen G hydrolytic activity was 1177 I.U./gww or 5600 I.U./gdw and 48.6% of the enzyme activity that was present in the original concentrated broth was retained in the insolubilized form.

Example 5

Reuse of insolubilized penicillinamidase for the hydrolysis of benzylpenicillin

Insolubilized penicillinamidase was produced in a manner similar to that described in Example 4. Benzylpenicillin was hydrolyzed in a reaction mixture consisting of 10% (w/v) insolubilized penicillinamidase, 5% (w/v) Celite, and 6% (w/v) benzylpenicillin using a stirred cell reactor. pH was maintained at 7.5 by the addition of $NH_4OH$ and the reaction temperature was kept at 37 degrees C. After the completion of each conversion, the liquid was separated from the insolubilized enzyme through a filter in the bottom of the stirred cell and fresh benzylpenicillin was added to the insolubilized enzyme which remained in the reactor. The percent of the initial benzylpenicillin hydrolyzed for 5 consecutive conversions (as determined by titration with $NH_4OH$) is listed in Table 3.

TABLE 3

% Conversion (by $NH_4OH$ titration)

| Run No. | 10 min | 15 min | 30 min | 45 min | 60 min |
|---------|--------|--------|--------|--------|--------|
| 1 | 20.4 | 32.6 | 68.0 | 91.5 | 100.9 |
| 2 | 60.2 | 79.0 | 99.1 | 100.9 | 101.3 |
| 3 | 56.7 | 74.9 | 98.9 | 100.9 | 101.6 |
| 4 | 53.3 | 71.5 | 95.9 | 100.0 | 100.9 |
| 5 | 51.1 | 69.6 | 94.7 | 99.1 | 100.0 |

Example 6

Stability of insolubilized penicillinamidase

Penicillinamidase was insolubilized as described in Example 4. The enzyme was suspended in either $0.05M$ TES [N-Tris-(hydroxymethyl)methyl-2-aminoethanesulfonic acid], pH 7.6 or $0.05M$ sodium tetraborate, pH 8.8 at a concentration of 10 g wet weight/100 ml buffer and placed into Erlenmeyer flasks. The flasks were shaken in a rotary shaker at 250 rpm for 1500 hours and assayed for benzylpenicillin hydrolytic activity as described previously. The percent retention of the initial activity is tabulated in Table 4.

TABLE 4

| Temp | pH | % retention of initial hydrolytic activity |
|------|-----|-------------------------------------------|
| 37 C | 7.6 | 100% |
| 37 C | 8.8 | 82% |
| 45 C | 7.6 | 86% |
| 45 C | 8.8 | 56% |

Example 7

Production of 6-aminopenicillanic acid by the hydrolysis of benzylpenicillin with insolubilized penicillinamidase

Insolubilized penicillinamidase was produced as described in Example 4. 675 g wet weight of enzyme with an activity of 1048.3 I.U./gww was suspended in a total of 15 liters of water containing 9% (w/v) benzylpenicillin (conversion grade) to give a final enzyme concentration of 48.1 I.U./ml and a substrate to wet enzyme ratio of 1:0.5. The hydrolysis reaction was performed at 42 deg. C with the pH kept constant at 8.1 by the addition of 3N $NH_4OH$. The reaction was stopped after 35 minutes at which time 96.2% of the theoretical 6-APA was produced as determined by high performance liquid chromatography.

Two liters of the filtered conversion broth were chilled in an ice bath. To this was added 1330 ml of chilled methanol while the temperature was maintained at less than 10 deg. C. The pH of the mixture was reduced to 5.0 with HCl and held at this pH for 20 min. The solution was then sequentially reduced to pH 4.8, 4.6, 4.4, and 4.2 and held at these values for 10 min between each decrement. After allowing crystallization for 2 hours at pH 4.2, the 6-APA crystals were harvested by filtration, washed with 100% methanol and

6

dried at 50 deg. C. 88.8 g of 6-APA crystals were recovered with a purity of 99.9% giving a yield of 91.1% of the theoretical yield.

Example 8
Hydrolysis of benzylpenicillin with insolubilized penicillinamidase

Insolubilized penicillinamidase was produced as described in Example 4. 1350 g wet weight of enzyme with an activity of 1048.3 I.U./gww was suspended in 15 liters of water containing 9% (w/v) benzylpenicillin (Conversion grade) to give a final enzyme concentration of 99.7 I.U./ml and a substrate to wet enzyme ratio of 1:1. The hydrolysis reaction was performed at 37 deg. C and the pH was kept constant at 7.5 by the addition of $3N$ $NH_4OH$. The reaction was stopped after 73 minutes at which time 89.4% of the theoretical 6-APA was produced as determined by high performance liquid chromatography.

**Claims**

1. An immobilized, intermolecular adduct of the exocellular penicillinamidase produced by *Bacillus mageterium* and an aliphatic dialdehyde containing from 5 to 7 carbon atoms.

2. An adduct as claimed in Claim 1 in which the dialdehyde is glutaraldehyde.

3. A process for the preparation of a penicillinamidasedialdehyde adduct which comprise adding an aliphatic dialdehyde containing 5 to 7 carbon atoms to an aqueous solution containing the exocellular penicillinamidase produced by *Bacillus megaterium,* at a pH of between about 6 to about 9.5 to obtain a final dialdehyde concentration of about 0.1 to about 2.0 weight/volume percent, at a temperature of from 0 to about 45°C., followed by the addition of a multivalent anion containing salt.

4. A process as claimed in Claim 3 in which the dialdehyde is glutaraldehyde.

5. A process as claimed in Claim 3 or 4 in which the multivalent anion is the phosphate, thiosulfate, borate, carbonate or sulfate ion in alkali metal or ammonium salt form.

6. A process for the preparation of 6-aminopenicillanic acid, 7-aminocephalosporanic acid or 7-aminodesacetoxycephalosporanic acid by incubating an aqueous solution of a natural penicillin or a cephalosporin containing a 7-acyl group corresponding to the 6-acyl group of a natural penicillin, with a penicillinamidase adduct characterised in that the penicillinamidase adduct is that claimed in Claim 1 or Claim 2.

7. A process as claimed in Claim 6 in which the penicillin is benzylpenicillin or phenoxymethylpenicillin or the cephalosporin is benzylcephalosporin C or phenoxymethyl-3-desacetoxycephalosporin.

8. A process as claimed in Claim 6 or 7 in which the products of the incubation are separated from the adduct with an anion exchange resin.

**Patentansprüche**

1. Immobilisiertes, intermolekulares Addukt der durch das Bazillus megaterium produzierten exocellulären Penicillinamidase mit einem aliphatischen Dialdehyd, der 5 bis 7 Kohlenstoffatome enthält.

2. Addukt nach Anspruch 1, bei dem der Dialdehyd Glutaraldehyd ist.

3. Verfahren zur Herstellung eines Penicillinamidase-Dialdehyd-Addukts, wobei man einen aliphatischen Dialdehyd, der 5 bis 7 Kohlenstoffatome enthält, zu einer wäßrigen Lösung, welche die durch das Bacillus megaterium produzierte exocelluläre Penicillinamidase enthält, bei einem pH-Wert von etwa 6 bis etwa 9,5 bis zu einer Dialdehyd-Endkonzentration von etwa 0,1 bis etwa 2,0 Gew./Vol.-%, bei einer Temperatur von 0 bis etwa 45°C zugibt und anschließend ein Salz mit einem multivalenten Anion hinzufügt.

4. Verfahren nach Anspruch 3, wobei man als Dialdehyd Glutaraldehyd einsetzt.

5. Verfahren nach Anspruch 3 oder 4, wobei man als multivalentes Anion das Phosphat-, Thiosulfat-, Borat-, Carbonat- oder Sulfation in Form eines Alkalimetall- oder Ammonium-salzes einsetzt.

6. Verfahren zur Herstellung von 6-Aminopenicillansäure, 7-Aminocephalosporansäure oder 7-Aminodesacetoxycephalosporansäure durch Inkubation einer wäßrigen Lösung Lösung eines natürlichen Penicillins oder eines Cephalosporins, das eine 7-Acylgruppe enthält, welche der 6-Acylgruppe des natürlichen Penicillins entspricht, mit einem Penicillinamidaseaddukt, dadurch gekennzeichnet, daß man das Penicillinamidaseaddukt gemäß Anspruch 1 oder gemäß Anspruch 2 einsetzt.

7. Verfahren nach Anspruch 6, wobei man als Penicillin Benzylpenicillin oder Phenoxymethylpenicillin oder als Cephalosporin Benzylcephalosporin C oder Phenoxymethyl-3-desacetoxy-cephalosporin einsetzt.

8. Verfahren nach Anspruch 6 oder 7, wobei man die Produkte der Inkubation von dem Addukt mit einem Anionenaustauscherharz abtrennt.

**Revendications**

1. Produit d'addition intermoléculaire immobilisé de la pénicillinamidase exocellulaire produite par *Bacillus megaterium* et d'un dialdéhyde aliphatique contenant 5 à 7 atomes de carbone.

2. Produit d'addition suivant la revendication 1, dans lequel la dialdéhyde est le glutaraldéhyde.

3. Procédé de préparation d'un produit d'addition pénicillinamidase-dialdéhyde qui consiste à ajouter un dialdéhyde aliphatique contenant 5 à 7 atomes de carbone à une solution aqueuse contenant la pénicillinamidase exocellulaire produite par *Bacillus megaterium*, à un pH d'environ 6 à environ 9,5, pour obtenir une concentration finale en dialdéhyde d'environ 0,1 à environ 2,0% en poids/volume, à une température de 0 à environ 45°C, puis à ajouter un sel contenant un anion multivalent.

4. Procédé suivant la revendication 3, dans lequel le dialdéhyde est le glutaraldéhyde.

5. Procédé suivant la revendication 3 ou 4, dans lequel l'anion multivalent est l'ion phosphate, thiosulfate, borate, carbonate, ou sulfate, sous forme de sel de métal alcalin ou de sel d'ammonium.

6. Procédé de préparation d'acide 6-amino-pénicillanique, d'acide 7-aminocéphalosporanique ou d'acide 7-aminodésacétoxycéphalosporanique par incubation d'une solution aqueuse d'une pénicilline naturelle ou d'une céphalosporine contenant un groupe acyle en position 7 correspondant au groupe acyle en position 6 d'une pénicilline naturelle, avec un produit d'addition de pénicillinamidase, caractérisé en ce que le produit d'addition de pénicillinamidase est celui revendiqué dans la revendication 1 ou la revendication 2.

7. Procédé suivant la revendication 6, dans lequel la pénicilline est la benzylpénicilline ou la phénoxyméthylpénicilline, et la céphalosporine est la benzylcéphalosporine C ou la phénoxyméthyl-3-désacétoxycéphalosporine.

8. Procédé suivant la revendication 6 ou 7, dans lequel les produits de l'incubation sont séparés du produit d'addition avec une résine échangeuse d'anions.